# EUROPEAN PATENT SPECIFICATION

(11) **EP 2 320 965 B1**
(45) Date of publication and mention of the grant of the patent: **20.03.2013**
(21) Application number: 09756807.5
(22) Date of filing: 12.10.2009
(51) Int. Cl.: A61L 27/34, A61L 29/08, A61L 31/10

(54) **IMPLANT DEVICE**
IMPLANTATVORRICHTUNG
DISPOSITIF D'IMPLANT

(30) Priority: 20.08.2008 IT MI20081525
(43) Date of publication of application: 18.05.2011
(73) Proprietor: Nobil Bio Ricerche S.r.l., 25062 Concesio (BS) (IT)
(72) Inventor: MORRA, Marco, I-14018 Villafranca D'Asti (IT); CASSINELLI, Clara, I-14018 Villafranca D'Asti (IT); CASCARDO, Giovanna, I-14018 Villafranca D'Asti (IT); BOLLATI, Daniele, I-14018 Villafranca D'Asti (IT)
(74) Representative: Long, Giorgio
(86) International application number: PCT/IB2009/054477
(87) International publication number: WO 2010/020972

(56) References cited:
- WO-A-2005/032417
- WO-A-2006/038056
- WO-A-2008/076731
- S.IBRAHIM ET AL.: "Hyaluronic acid cues for functional endothelialization of vascular constructs" JOURNAL OF TISSUE ENGINEERING AND REGENERATIVE MEDICINE, vol. 2, 11 February 2008 (2008-02-11), pages 22-32, XP002550483
- M.MORRA: "Engineering of Biomaterials Surfaces by Hyaluronan" BIOMACROMOLECULES, vol. 6, 2005, pages 1205-1223, XP002550484
- M.MORRA ET AL.: "Effects of molecular weight and surface functionalization on surface composition and cell adhesion to Hyaluronan coated titanium" BIOMEDICINE & PHARMACOTHERAPY, vol. 60, 2006, pages 365-369, XP002550485
- MARCO MORRA: "Biomolecular modification of implant surfaces" EXPERT REVIEW OF MEDICAL DEVICES, FUTURE DRUGS LTD., LONDON, GB, vol. 4, no. 3, 1 January 2007 (2007-01-01) , pages 361-372, XP009098770 ISSN: 1743-4440
- MORRA M ET AL: "Biomaterials surface characterization and modification" INTERNATIONAL JOURNAL OF ARTIFICIAL ORGANS, MILAN, IT, vol. 29, no. 9, 1 September 2006 (2006-09-01), pages 824-833, XP008081984 ISSN: 0391-3988

## Description

The present description relates to an implant device, having an improved integration in the tissue of an animal or human body into which it is inserted as compared with prior art devices.

### Background of the invention

The insertion of an implant device in a human body necessarily involves a wound and the consequent tissue healing process. The healing of tissues, in nature, occurs through the natural mechanism of inflammation and healing of the wound. In the presence of an implant device, the natural inflammation process can be altered, due to the chronic presence of what is perceived as a foreign body, giving rise to harmful outcomes for the functionality of the device itself and health. The inflammation and healing mechanism is mediated by the inflammatory cells, mainly neutrophils and macrophages. These cells reach the wound site and process and emit molecules, defined as cytokines and chemokines, which call other inflammatory cells and stimulate the activity thereof. In normal tissue healing, the cytokines and chemokines produced by the inflammatory cells appear in precise sequences and temporal concentrations, directing the healing process. Some of these molecules have a pro-inflammatory effect, other ones have an anti-inflammatory effect. By appropriately balancing the necessary pro-inflammatory stimuli, for example, for the removal of damaged tissue, particulate, bacteria, and the anti-inflammatory ones, required to keep the process under control and avoid undue damages, our defence mechanisms normally manage to induce the tissue healing.

Under pathological conditions, or in the presence of implant devices, the normal production of pro- and anti-inflammatory stimuli by the corresponding cells may be unbalanced: for example, solid material particles (such as fragments of implant material), of a size on the order of the micron, which cannot be phagocyted by the inflammatory cells intended to the elimination thereof, stimulate the inflammatory cells themselves (macrophages). Under the influence of a foreign body (the particle) which the macrophage is not able to phagocyte, it emits further pro-inflammatory chemokines and cytokines, to call to the site other inflammatory cells to eliminate the external stimulus. As the stimulus (the non-phagocytable foreign material) continues, the process continues to repeat itself, giving rise to the chronic inflammation situations that can constitute a great source of problems, for example, for the orthopaedic devices.

In other cases, the unbalanced production of chemokines and cytokines may induce transformations and alterations in the cells that not allow the complete healing and lead to the formation of abnormal tissue. The typical case is represented by the formation of secondary restenotic tissue upon the introduction of coronaric or peripheral stents: it is by now proven that the restenosis is an inflammatory disease, due to the combination of a tissue insult (wound) of the cells of the vascular walls, the contact and the chronic presence of a foreign material (stent), besides to local alterations of fluidodynamics and mechanical properties of the vessel. The secretion altered by macrophages of cytokines and chemokines in the inflammatory site generated by the stent insertion causes an altered cell behaviour, which shows by a neo-tissue formation that reduces the useful section of the vessel. It is significant how the more recent researches have defined the restenotic tissue as an "incomplete healing" tissue, being composed of sparse cells in an environment of glycosaminoglycanes. During the normal healing, this type of tissue is an intermediate step, which is typically followed by the deposition of collagen from cells, with consequent contraction of the tissue itself. If this should occur in the restenotic tissue as well, a major part of the problems would be solved, since the contraction would lead to an increase of the vessel useful section. Unfortunately, the alteration of the inflammatory, and then healing, processes does not allow reaching a complete healing in this case, and restenosis continues to be a concern for interventional cardiology.

Under this point of view, it should be pointed out that, even if, in the common language, the term "inflammation" has a generally negative connotation, this is not true: "acute" inflammation, secondary to a wound, identified by the key signs of swelling, pain, flush, and heat, is the natural process required for the healing of the wound itself. The inflammatory activity presides over the removal of the damaged tissue of possible bacteria, and triggers the healing mechanisms. The inflammation cannot and must not be suppressed, or else, there would be no healing; the inflammation has to be controlled, as occurs in the natural processes, and as does not occur in the chronic inflammation diseases (ad es., rheumatoid arthritis), or not always occurs in the periimplant site following the insertion of an implant device.

The macrophages are often regarded as the key type of inflammatory cells, since, as reminded, process and secrete a great number of fundamental chemical stimuli for the correct course of the healing process. Consequently, a great deal of scientific knowledge has been developed about the behaviour of the macrophages in the normal inflammatory processes and in contact with implant materials. It is common sense that the ability to direct the production of cytokines and chemokines and, in general, the behaviour of macrophages at the interface with materials, would open new possibilities for the implant devices.

In nature, there is a key molecule controlling the behaviour of the macrophages in the inflammation processes: the hyaluronic acid. This molecule is present in all tissues, and is part of the extracellular matrix. Its role is such that it shows completely different behaviours according to the molecular weight thereof: the high molecular weight hyaluronic acid is anti-inflammatory, and under this form it is normally found in our tissues. On the contrary, low molecular weight hyaluronic acid has pro-inflammatory effects: in a number of chronic inflammation diseases, hyaluronic acid is depolymerized by specific enzymes or by substances and brought to molecular weights on the order of the hundreds or tens of thousands Da. Such fragments act directly on the macrophages, causing the stimulation of different genes delegated to the production of pro-inflammatory molecules.

Ibrahim S. Et al. ("Hyaluronic acid cues for functional endothelialisation of vascular constructs" J. Tissue Eng. Reg. Med. 2008; 2:22-32) describes the effects of low molecular weight hyaluronic acid and high molecular weight hyaluronic acid on vascular endothelialisation. Morra M. et al. ("Effects of molecular weight and surface functionalization on surface composition and cell adhesion to Hyaluronan coated titanium" Biomed. & Pharmacother. 2006; 60:365-369) describes the use of low molecular weight and high molecular weight hyaluronic acid separately on titanium surfaces. WO2006/038056 discloses implant devices coated by hyaluronic acid.

Morra M. ("Biomolecular modification of implant surfaces" Expert Rev. Med. Dev., Future Drugs Ltd., London, GB 2007; 4:361-372) and Morra M. et al. ("Biomaterials surface characterization and modification" Int. J. Artificial Organs, Milan, IT 2006; 29:824-833 review the field of biomolecular modification of surfaces and Morra M. (Engineering of biomaterials surfaces by hyaluronan" Biomacromolecules 2005: 6:1205-1223) reviews existing approaches to surface modification by hyaluronic acid.

### Summary of the invention

The object of the present description is to provide an implant device in the human body that is provided with high biointegration.

Such object is achieved by means of an implant device as set forth in the annexed claims, the definitions of which form an integral part of the present description.

The present description is based on the evidence that it is possible to replicate the natural control mechanism of the inflammation on implant devices, so as to stimulate the inflammation, and therefore to promote healing, in the sites where the device does not lead to a complete healing; to promote the production of anti-inflammatory cytokines in the sites in which the inflammation tends to become chronic; or to modulate the production of cytokines essential for the correct course, but produced in an incorrect manner in the periimplant site.

The present description originates from the surprising finding that it is possible to transform a material so as to affect the production of inflammatory molecules by macrophages, chemically linking hyaluronic acid of different molecular weight to the surface of a polymeric, metallic, or ceramic device, so as to affect the activation thereof of the genes of macrophages delegated to the production of inflammatory molecules. We have observed, which could not have been foreseen in advance, that the molecular weight of hyaluronic acid has an effect on the inflammatory cells also when this molecule is stably linked (covalent linkage) to a surface. In fact, it has been observed that a given molecular weight of hyaluronic acid linked to the surface induces in macrophages the expression of inflammatory genes that are different from those expressed by the same cells in contact with hyaluronic acid with different molecular weight. We noticed that this activity is mouldable by acting on the molecular weight of hyaluronic acid and by operating, with different molecular weights, on the relative amount thereof. In general, the low molecular weights induce more inflammatory molecules compared to high molecular weights.

The implant devices and the method for the preparation thereof that are the object of the present description provide for the coating of such devices with molecules of hyaluronic acid with controlled molecular weight, thus exerting, according to the cases, pro- or anti-inflammatory effects on macrophage cells; these effects, embodied in the production of chemokines and cytokines as a function of the molecular weight of surface-linked hyaluronic acid, alter the periimplant environment and affect the healing process; this allows, in principle, selecting the molecular weight of hyaluronic acid, or the weight combinations, as a function of the disease that can be induced by the implant device. In the case of devices that cause an excessive inflammation, a high molecular weight anti-inflammatory effect can be used. In the case in which a stimulation of the healing is required, a low weight or a combination of weights can be used.

### Brief description of the Figures

Fig. 1 is a graph showing the number of macrophages adhered on an untreated or treated steel substrate according to the invention;
Fig. 2 is a graph showing the number of macrophages adhered on an untreated or treated substrate in titanium according to the invention;
Fig. 3 is a graph showing the gene expression of two key cytokines at 24 h in Macrophages J774A.1 cultured on steel discs treated with HA;
Fig. 4 is a graph showing the gene expression of two key cytokines at 24 h in Macrophages J774A.1 cultured on steel discs treated with different MW of HA;
Fig. 5 is a graph showing the gene expression of protein MCP-1 in macrophages cultured on steel discs treated with HA according to the invention;
Fig. 6 is a graph showing the gene expression of key protein in macrophages cultured on treated polystyrene according to the invention.

### Description of the invention

The object of the present description is an implant device, according to claim 1.

The implant device according to the invention can be made of metal (for example, steel, titanium or their alloys with other metals) or in plastic material (such as, for example, polystyrene), compatible for applications on the animal or human body.

In a particularly advantageous embodiment of this invention, the device is composed of a screw for dental implant, preferably in titanium or alloys thereof, optionally of the transmucosal type, or of a screw, preferably in titanium or alloys thereof, for spinal or skeletal fixing, or by an intervertebral disc, preferably in titanium, alloys thereof or chromium-cobalt alloys or in the metal alloys commonly used for these applications, or by a stent, of the coronary or peripheral type, optionally a "drug eluting stent" (DES), preferably in titanium or alloys thereof, or by a balloon for catheters, in suitable plastic/elastomeric material.

The hyaluronic acid is immobilized to the surface of the device in a thin layer, preferably from 0.5 to 10000 nm, more preferably between 1 and 1000 nm, still more preferably between 1.5 and 100 nm.

The process of immobilization of hyaluronic acid on an implant device according to the invention provides for the introduction of amino functional groups on the device surface and the consequent linkage of hyaluronic acid to said amino groups.

The substrate containing the amino groups can be deposited on the surface of the implant device according to methods widely known in the art. The technique providing for the introduction of the substrate having amino functional groups on the surface of the implant device by plasma deposition of molecules containing amino groups is particularly advantageous. Typical examples of molecules used to this aim are allylamine, alkylamines such as hexyl or heptyl amine and, in general, organic molecules with amine functionality that have the required volatility characteristics in the plasma phase. The deposition of the amine from plasma occurs under the following conditions: pressure ranging between 80 and 300 mTorr, discharge power ranging between 5 and 200 W, deposition time between 1 ms and 300 s. Plasma deposition can also occur under pulsed plasma conditions, with active and inactive plasma cycles ranging between 1 and 100 ms, to minimize the molecular fragmentation and maintain the highest density of amino groups possible. The plasma deposition treatment of the amine can be preceded by other treatments with plasma, for example, with air or oxygen plasma to clean the surface and increase the adhesion with the substrate.

A further method for the coating of the implant device with a substrate containing amino groups consists in the adsorption on the device surface of polyethyleneimine (PEI), for example, from 0.2% aqueous solution, for 2 h, at room temperature.

A further method for the coating of the implant device with a substrate containing amino groups consists in the adsorption of collagen, preferably collagen of type I, from an aqueous solution.

In this case, the collagen is irreversibly adsorbed at the surface and provides the amino groups required for the successive hyaluronic acid linkage. This functionalization method makes possible to combine the properties of collagen, for example, the ability to accelerate the bone healing processes by the action on the osteogenic cells, and hyaluronic acid.

The hyaluronic acid is covalently linked to the amined surface, obtained as described before, by known and conventional methods of the state of the arte.

Preferably, such functionalization occurs by the reaction between amino groups of the surface substrate and carboxy groups of hyaluronic acid promoted by suitable condensing agents that are known to those skilled in the art. A carbodimiide can be advantageously used, particularly 1-ethyl-3-[3-dimethylaminopropyl]carbodimiide hydrochloride (EDC) and n-hydroxysuccinimide (NHS). The reaction can be carried out by adding EDC and NHS to an aqueous solution of hyaluronic acid at concentrations between 0.3 and 0.5%, at room temperature, for a period of time ranging between 2 and 24 h. It is suitable to use apyrogen and sterilized water.

The hyaluronic acid can also be linked by the hydroxyl groups thereof to the amine layer from aqueous solution or of suitable solvents, such as dimethylsulphoxide or mixtures thereof with water, dimethylformamide or mixtures thereof with water, N-methyl pyrrolidone or mixtures thereof with water, by the methods that are known in the art for the functionalization of hydroxyl groups, and particularly for the substitution of a hydroxyl group with a linkage of the amine type:

Ial-OH + Sub-NH₂ -> Ial-NH-Sub

where Ial is the residue of hyaluronic acid and Sub is the residue of the substrate having amine functionalities. From what has been stated above, it shall be apparent that the method of the invention will be able to provide for the functionalization of all reactive hydroxyl groups of the hyaluronic acid, as well as a part thereof, according to the used reaction and the conditions of reaction applied from case to case. However, it is necessary and sufficient that the functionalization reaction of the hyaluronic acid hydroxyl groups leads to the formation of a linked hyaluronic acid layer with a fractional coating higher than 0.6, meant as the fraction of the surface that is coated by hyaluronic acid, which can be assessed by a surface chemical composition analysis.

The functionalization reaction of the hyaluronic acid hydroxyl groups with the substrate amine will be able to be carried out according to various methods that are known to those skilled in the art, such as the following ones (listed by way of non-exhaustive example):
- activation of the hydroxyl group by formation of mesylates, tosylates, or analogous leaving groups, for example, by reaction of hyaluronic acid with mesyl or tosyl chloride, and successive reaction of the hydroxyl groups activated with amine;
- substitution of the hydroxyl group with a halogen, such as chlorine, bromine, or iodine, for example, by reaction of hyaluronic acid with thionyl chloride or with carbon tetrabromide and triphenylphosphine, and then of halogenated hyaluronic acid with amine;
- Mitsunobu reaction of hyaluronic acid with amine, in the presence of diethylazadicarboxilate and triphenylphosphine;
- oxidation of primary hydroxyl groups to aldehydes, and successive reducing amination.

The object of the invention is an implant device in the animal or human body coated with a mixture of low molecular weight hyaluronic acid, i.e., having molecular weight lower than 80,000 Dalton, with high molecular weight hyaluronic acid, i.e., having molecular weight higher than 700,000 Dalton.

Preferably, such low molecular weight hyaluronic acid, has a molecular weight lower than 30,000 Dalton, more preferably lower than 19,000 Dalton, still more preferably lower than 15,000 Dalton, and most preferably lower than 10,000 or than 7,000 Dalton and higher than 1,000 Dalton.

Preferably, such high molecular weight hyaluronic acid has a molecular weight higher than 1,000,000 Dalton, more preferably higher than 1,500,000 Dalton, still more preferably higher than 2,000,000 Dalton and lower than 3,000,000 Dalton.

In a preferred embodiment, the percentage of low molecular weight hyaluronic acid (LMW HA) relative to the total hyaluronic acid (high + low molecular weight) on the implant, will range in the following ranges of weight percentages (the remaining percentage is constituted by high molecular weight hyaluronic acid, HMW HA):
10% to 90% LMW HA, preferably 20% to 80%, more preferably 25% to 75%, still more preferably 50% to 65%; or
10% to 50% LMW HA, preferably 15% to 45%, more preferably 25% to 40%.

The selection of the optimal ratio between the low MW component and the high MW component depends on the type of implant which is desired to implement, and particularly on the use thereof. In fact, such embodiment allows combining the advantages of the stimulation of the inflammatory response (by means of LMW HA) with those of the containment of the same (by HA HPM), by modulating such effects, according to the needs, simply by varying the ratio between the two components and/or selecting the optimal molecular weights of hyaluronic acid.

In an embodiment, an orthopaedic implant such as an osteo-implant on elderly patients, characterized by a tissue with poor tendency to heal, the healing process will be able to be accelerated by using on the implant a mixture in which LMW HA is present in amounts ranging between 20% and 30% by weight, and HMW HA, consequently, is present in amounts ranging between 80% and 70% by weight, and in which LMW HA has a molecular weight ranging between 10,000 and 15,000 Dalton and HMW HA has a molecular weight ranging between 700,000 and 1,000,000 Dalton.

In a second embodiment, a vascular stent will be able to comprise a coating in which LMW HA is present in amounts ranging between 35% and 45%, while HMW HA is present in amounts ranging between 65% and 55% by weight, and in which LMW HA has a molecular weight ranging between 30,000 and 80,000 Dalton and HMW HA has a molecular weight higher than 2,000,000 Dalton and lower than 3,000,000 Dalton.

In this manner, the healing of the tissue around the stent can be obtained, since it is more stimulated, without falling in the chronic inflammation phenomena due to the excessive production of pro-inflammatory cytokines.

The implant devices comprising a coating formed by a mixture of LMW HA and HMW HA as described herein above can be implemented with the methods described above in relation to the implant device coated with LMW HA alone.

In an embodiment, a vascular stent as defined above will be able to be produced by means of a method providing for:
a) incubation of the stent in a polyethyleneimine aqueous solution for a preset period of time, preferably ranging between 1 hr and 3 hr at room temperature;
b) incubation of the treated stent according to step a) in an aqueous solution of low molecular weight hyaluronic acid, as defined above, in the presence of a hydroxyl group activator as defined above and for a preset period of time;
c) incubation of the treated stent according to step b) in an aqueous solution of high molecular weight hyaluronic acid, as defined above, in the presence of a hydroxyl group activator as defined above and for a preset period of time.

The steps b) and c) can also be reversed, this meaning that the stent treated according to step a) can be first incubated in the HMW HA solution and then in the LMW HA solution.

The incubation times can be varied in order to modify the percentage of LMW HA or HMW HA that is present on the stent surface. In an embodiment, such incubation times are 1-3 hr for LMW HA and 13-18 hours for HMW HA, operating at room temperature.

The hydroxyl group activator, in an embodiment, is a mixture of a carbodiimide and N-hydroxysuccinimide.

In the above-described embodiments, LMW HA will preferably have a molecular weight ranging between 1,000 and 10,000 Dalton and HMW HA will preferably have a molecular weight ranging between 1,500,000 and 3,000,000 Dalton.

The invention will be now further described by means of some implementation examples, which are anyhow not intended as a limitation of the invention as defined in the annexed claims.

In the following implementation examples and in the relative experimental biological section, samples of hyaluronic acid were used as defined in the following table I:

**TABLE I**

| **Definition** | **Our code** | **Batch number** | **Average molecular weight (Da)** | **Endotoxins** |
|---|---|---|---|---|
| LMW HA | L | GSP252-5-2 | 6.5 x 10³ | 0.008 EU/mg |
| LMW HA | L2 | GSP252-10-2 | 1.2 x 10⁴ | < 0.002 EU/mg |
| LMW HA | M | GSP252-60-2 | 7.6 x 10⁴ | < 0.01 EU/mg |
| HMW HA | H | 014591 | 7.3 x 10⁵ | < 0.01 EU/mg |
| HMW HA | HH | 012424 | 2.6 x 10⁶ | < 0.02 EU/mg |

The batch number relates to the batch of the supplier Lifecore Biomedical 3515 Lyman Blvd, Chaska, MN, USA. HA was obtained by bacterial fermentation.

The effect of the molecular weight of linked HA was assessed in experiments with cell cultures, using the continuous line of macrophages J774A.1, purchased at the Istituto Zooprofilattico Sperimentale of Brescia and cultured according to the known methods. Particularly, the macrophages were put in contact with the samples of materials on which HA with different molecular weight had been linked. The effect was assessed by direct microscopy observation of the cell behaviour, cell count, and gene expression measurements, as described in the following examples.

### Example 1 - Implant device coated with mixture of HA with different MW

A process of surface modification of a screw for orthopaedic implant in titanium was carried out by using a mixture of hyaluronic acid composed for 25% of HA L2 and for 75% of HA H. The implant screw was initially functionalized adsorbing porcine collagen type I (Symatese Biomateriaux) at 0.1% by 50% physiological solution and 50% acetic acid solution, overnight. After washing, the collagen adsorbed provides the surface amino groups necessary to link the hyaluronic acid. The implant screw was put in the solution of 0.5% hyaluronic acid composed of 25% of HA L2 and of 75% of HA H. 0.04 g of 1-Ethyl-3-[3-dimethylaminopropyl]carbodimiide hydrochloride (EDC) and 0.03 g N-hydroxysuccinimide (NHS) in 5 cc of solution were added. The reaction was carried out at room temperature, for 2 hours. The samples were put in contact with macrophage cells J774A.1 for 15 h, and the gene expression of MCP-1 was measured on the thus-modified screw, comparing it to an untreated screw and to one obtained by operating with 0.5% solution of HA H alone. The HA L2- and HA H-modified screw has expressed a gene coding for MCP-1 1.8 folds more than the untreated one. That obtained with HA H alone expressed 0.8 folds. The MCP-1 chemokine plays an important role in the healing processes: knockout mice for the gene that synthesizes MCP-1 showed a considerable delay in the healing of wounds. For orthopaedic applications, in tissue with poor respose, such as the tissue of elderly people, the high presence of MCP-1 in the wound site can involve an acceleration of the healing process.

### Example 2 - Implant device coated with mixture of HA with different MW

Samples of peripheral stent in chromium-cobalt alloy (Scuba, Invatec, Italy) are surface functionalized by dipping in 0.2% polyethyleneimine aqueous solution for 2 h. The samples are then immersed in the following solutions:
a) 100% HA M (Comparative example, not falling und a the scope of the invention)
b) 40% HA M + 60% HA HH
c) 100% HA HH (Comparative example, not falling und a the scope of the invention)
in all cases, the total HA concentration is 0.25%, and EDC and NHS are added in a proportion of 0.04 and 0.03 g for 5 cc of solution. After a night in solution, the samples are washed, put in contact with J774A.1 macrophage cells, and the gene expression is measured after 15 h of TNF α, IL-6 and IL-10 on samples of the three series, with reference to the unmodified stent. The following results are obtained:

| Sample (reference to the solution) | TNF α | I-6 | I-10 |
|---|---|---|---|
| a | 1.20 | 1.7 | 0.8 |
| b | 1.10 | 1.3 | 1.4 |
| c | 0.85 | 1.2 | 1.3 |

The example shows that the mixture induces a higher response in the expression of the anti-inflammatory interleukin IL-10 compared to HA M alone, in combination with a higher expression of the stimulation cytokine TNF α compared to HA HH. This characteristic could promote the healing of the tissue around the stent, since it is more stimulated, without falling in the chronic inflammation phenomena due to the excessive production of pro-inflammatory cytokines.

### Example 3 - Coated implant device with HA having a low MW (Comparative example, not falling und a the scope of the invention)

The surface of an intervertebral disc in chromium-cobalt (Link, Germany) is immersed in a 0.2% polyethyleneimine solution (Sigma) in water. After 2 hours, the device is washed, as it is known, the polyethyleneimine irreversibly links to the metal surfaces, providing a high density of amino groups. The device is immersed in a 0.5% HA M aqueous solution, in the presence of EDC and NHS. The sample is put in contact with macrophage cells J774A.1, and the gene expression for TNF α MCP-1 is measured after 15 h compared to the unmodified control. Both genes are expressed with an increase above 50% on the modified device. This effect can involve a higher and more efficient reparative response by the vertebral tissue.

### Example 4 - Multi-functionalized implant device (Comparative example, not falling und a the scope of the invention)

A screw for dental implant in titanium of the transmucosal type is surface functionalized by plasma deposition of allylamine. The portion, the use of which is provided for is the interface with the bone (threaded portion of the screw), is put in a 0.5% HA H solution, in the presence of EDC and NHS, for 12 h. The portion, the use of which is provided for is the mucosa interface is, in this step, shielded and not exposed to the solution. At the end of the reaction, the transmucosal portion alone is put in a 0.5% HA L solution, in the presence of EDC and NHS. In this manner, a multifunctional device is implemented, the transmucosal portion of which is stimulated to react, similarly to what has been observed in the presence of bacterial endotoxins (in the literature, it is shown that the Lipopolysaccharide (LPS) toxin induces a stimulation in the macrophages of genes analogous to those induced by low molecular weight HA, in the article Hyaluronan (HA) Fragments Induces Chemokine Gene Expression in Alveolar Macrophages, The Role of HA Size and CD44, C M. McKee, M B. Penno, M Cowman,M D Burdick, R. M. Strieter, C Bao, P W. Noble, J. Clin. Invest. Volume 98, Number 10, November 1996, 2403-2413). Therefore, the tissue put preventively into effect the defence mechanisms induced by the presence of bacterial toxins, without undergoing the negative consequences thereof.

### Example 6 - Implant device coated with mixture of HA with different MW

Samples of peripheral stent in chromium-cobalt alloy (Skylor, Invatec, Italy) are surface functionalized by dipping in 0.5% polyethyleneimine aqueous solution for 2 h at room temperature. The samples are then immersed in a 0.5% w/v LMW HA aqueous solution (4,300 Dalton), in the presence of 7.5 mg/ml N-(3-dimethylaminopropyl-N'-ethyl carbodiimide hydrochloride and 5 mg/ml N-hydroxysuccinimide for 2 hours at room temperature. The stents are then washed in water twice and dried, then they are immersed in a 0.4% w/v HPM (2,000,000 Dalton) HA aqueous solution in the presence of N-(3-dimethylaminopropyl-N'-ethyl carbodiimide hydrochloride and 5 mg/ml N-hydroxysuccinimide for 15 hours at room temperature. Finally, the stents are washed twice in water and dried.

The percentage of surface area coated with LMW HA resulted to be of about 50-60%, the remaining area being coated with HMW HA. The determination was performed by XPS (X-ray Photoelectron Spectroscopy) analysis.

### Example 7 - Assessment of the pro-inflammatory activity of HA at low MW on polystyrene substrate (Comparative example, not falling under the scope of the invention)

Surfaces modified by plasma deposition of allylamine on polystyrene for cell cultures were produced; particularly, 24-well microplates are used.

A column of 6 wells was modified by linking HA L, another one by linking HA H. Macrophages J774A.1 were cultured in the modified wells and in the controls. The cells were observed by optical microscopy. After 1 day, apparent signs of cell death of the macrophages grown on HA L were observed. The evidences are completely similar to those found when the macrophages are stimulated by powerful inflammatory agents, as the endotoxin LPS. The level of endotoxins of the original HA, as reported in Table I, is analogous, and cannot account for the difference. This experiment suggests that HA L, linked to the surface of a solid material, indicates a considerable pro-inflammatory effect, unlike HA H.

### Example 8 - Assessment of the pro-inflammatory activity of low MW HA on metallic substrate (Comparative example, not falling under the scope of the invention)

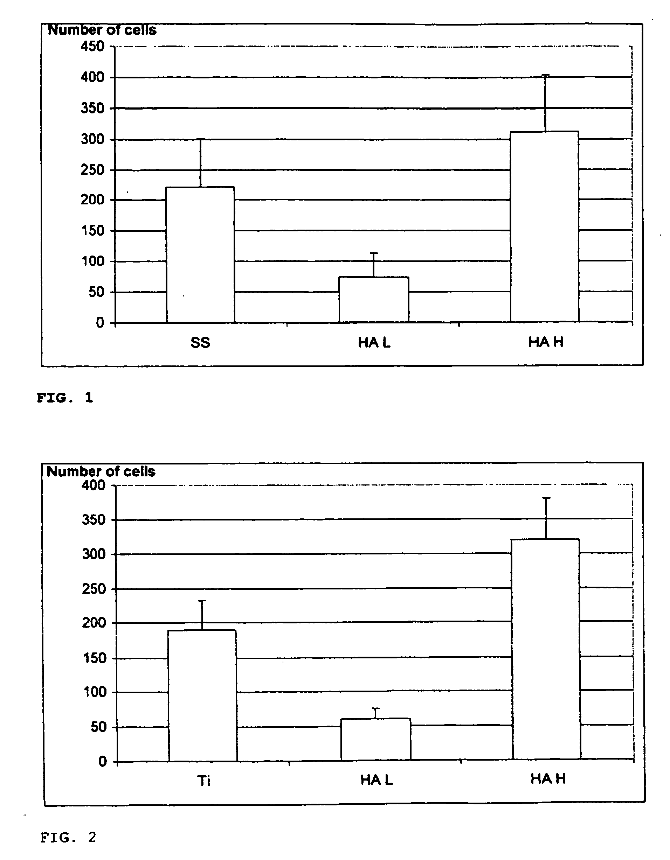

The experiment of the example 6 was carried out on AISI 304 steel (abbreviated as SS) discs. The macrophages were cultured for 2 days, then the number of adhered cells was assessed by fluorescence microscopy. The obtained results are reported in Fig. 1.

The data indicate that significantly less macrophages are present on HA L, confirming the toxic phenomena induced by this molecular weight. It shall be noted that the effect is specific for inflammatory cells, since for cells of the osteoblastic, fibroblastic type, or anyway for tissue cells, this effect does not occur, as reported in, for example: Morra M, Cassinelli C, Carpi A, Giardino R, Fini M., Effects of molecular weight and surface functionalization on surface composition and cell adhesion to Hyaluronan coated titanium, Biomed Pharmacother. 2006 Sep; 60(8):365-9. The observation that specific phenomena for inflammatory cells occur on HA covalently linked to surfaces is surprising per se and not foreseeable by the knowledge about general cell cultivation.

The same experiment was repeated on titanium discs. The trend is perfectly overlappable, as shown in Fig. 2, and demonstrates that the effect is not related to the material that is used as the substrate.

As it has been reminded, these results suggest that HA linked to the surface of materials, and then to implant devices, can induce a different inflammatory response as a function of the molecular weight thereof. Consequently, the inflammatory cells should produce different cytokines/chemokines, creating a different periimplant environment, and therefore affecting the cell recruiting at the wound site and, ultimately, the healing process.

### Example 9 - _Production of cytokines/chemokines (Comparative example, not falling und a the scope of the invention)

In order to verify the hypothesis of a cytokines/chemokines production that can be affected by the molecular weight of HA linked to implant devices, experiments were carried out by RT-PCR (Real Time Polymerase Chain Reaction) measurements. In these experiments, the expression of genes coding for some cytokines/chemokines was measured, by extracting the messenger RNA (mRNA) of macrophages exposed to solid samples coated with HA of different molecular weight. Particularly, the experimental scheme was as follows:
a. The cell culture medium was withdrawn from the wells containing the samples so as to analyze only the adhered cells;
b. The samples were treated with a solution of cell lysis, and then mRNA was extracted by means of a specific commercial kit (Ambion);
c. The obtained mRNA was subsequently retrotranscribed (i.e., converted into cDNA by a commercial kit) in order to obtain a more stable nucleic acid, and moreover a double strand, thus useful in the successive PCR reaction;
d. cDNAs, combined with the Taq polymerase enzyme and fluorescent probes specific for the genes of interest, were subjected to the PCR (Polymerase Chain Reaction) reaction. The protocol provides for 40 thermal cycles, each of which consisting in two steps: the first one, termed denaturation, at a temperature of 95 °C, the second one, termed annealing-extension, at a temperature of 65 °C.

The steps c-d and the reading of the results were carried out by an Applied Biosystems Step One apparatus.

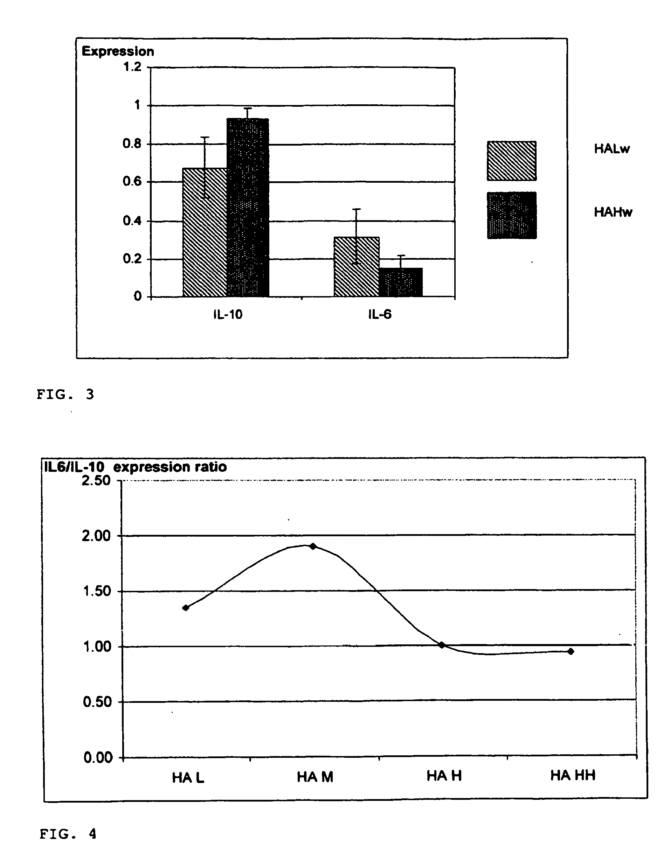

Steel discs were prepared by adsorption of PEI and successive linking of HA L and HA H. Macrophages J774A.1 were cultured on these samples, and the gene expression of two key cytokines was measured at 24 h. The obtained results are reported in Fig. 3.

Fig. 3 shows the inversion of the relationship between the expression of interleukin 10 (IL-10) and interleukin 6 (IL-6) as a function of the molecular weight, with statistically significant variations. As it is known, IL-10 has an anti-inflammatory effect, while IL-6 has a pro-inflammatory effect. The difference between the ratios of the two cytokines as a function of the molecular weight indicates that the macrophages on HA L are creating a pro-inflammatory environment, confirming the previous results; instead, the macrophages on HA H are more markedly producing a molecule (IL-10) that contributes to the inflammation containment. Also other genes coding for relevant protein molecules (TNFα, MCP-1) are expressed in a significantly different manner.

### Example 10 - Production of cytokines/chemokines with HA with different MW (Comparative example, not falling und a the scope of the invention)

The same experiment of the example 8 was repeated using different molecular weights of HA. The ratios IL-10/IL-6 obtained are shown in Fig. 4.

This graph shows that, in a manner that is not foreseeable by the current knowledge, the definition "HA-based coating" has not an unambiguous meaning, as regards the production of the molecules controlling the healing processes. With apparent practical implications, the periimplant environment around a device coated with HA L or, particularly, HA M, will have a ratio of IL-6/IL-10 gene expression opposite as HA H or HA HH. A HA L-based coating is not equivalent to a generic HA coating, and will be superior to a coating with HA HH for the cases in which a response of the defence mechanisms is intended to be stimulated. On the contrary, in order to try to reduce the production of pro-inflammatory cytokines such as IL-6, a coating with HA HH or HA H will be superior to coatings containing lower molecular weights.

### Example 11 - Expression of the gene for MCP-1 (Comparative example, not falling und a the scope of the invention)

In a further experiment, carried out on AISI 316 L steel discs, used for the implementation of coronary stents, the expression of the gene coding for the Monocyte Chemoattractant Protein 1 (MCP-1) was assessed. As described in the literature, this protein plays a key role in the restenosis phenomena (Essential Role of Monocyte Chemoattractant Protein-1 in Development of Restenotic Changes (Neointimal Hyperplasia and Constrictive Remodeling) After Balloon Angioplasty in Hypercholesterolemic Rabbits Mori E, Komori K, Yamaoka T, Tanii M, Kataoka C, Takeshita A, Usui M, Egashira K, Sugimachi K, Circulation. 2002; 105:2905-2910).

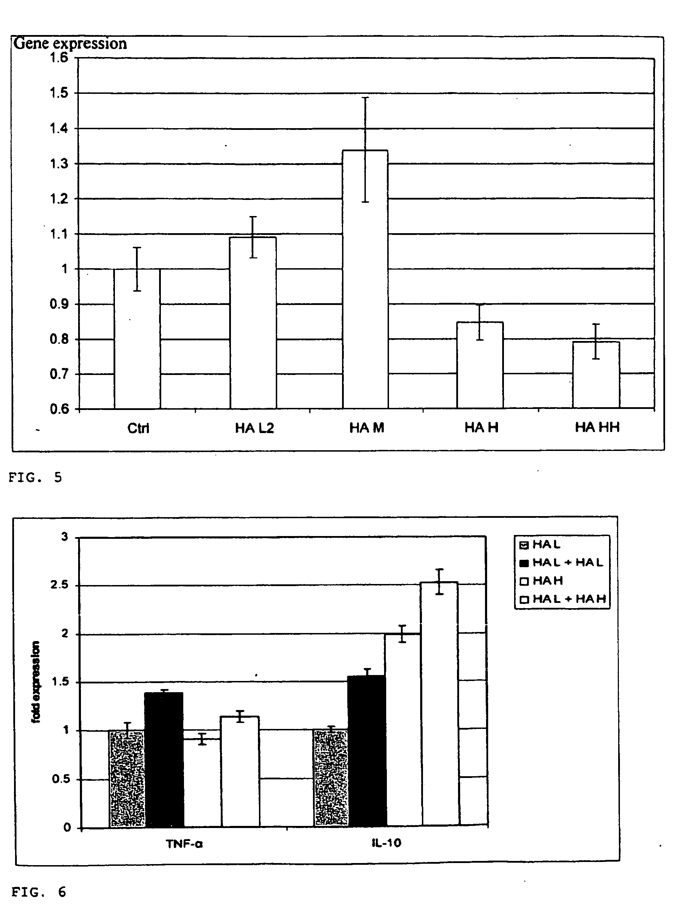

As it shall be appreciated from Fig. 5, the gene expression of this chemokine is completely different according to the type of HA linked to the surface, reaching a peak for molecular weights of HA ranging between 30,000 and 80,000 Dalton.

### Example 12 - Functionalization of polystyrene with HA at various MW

The use of mixtures of HA with different molecular weight leads to different results compared to what can be obtained with only one molecular weight. In these experiments, HA of different weights was linked on polystyrene in two successive steps. The results were assessed by RT-PCR, and are shown in Fig. 6.

In substance, the use of different molecular weights of HA, or combinations thereof, leads to results that cannot be captured by the simple definition of "coating with HA". The use of HA with different molecular weights can be advantageously exploited as a function of the implant device. In the graph, HAL + HAL is reported: it is a sample obtained by repeating the step of linking HAL twice on the amined substrate (Comparative example, not falling und a the scope of the invention).By repeating such step, still available amino groups can be saturated, thereby increasing the surface density of HA.

A further object of the invention is the use of a mixture of hyaluronic acid with molecular weight lower than 80,000 Dalton as defined herein above and hyaluronic acid with molecular weight higher than 700,000 Dalton or higher than 1,000,000 Dalton or higher than 1,500,000 Dalton or higher than 2,000,000 Dalton and lower than 3,000,000 Dalton, in the preparation of an implant device on which said hyaluronic acid is immobilized or of a medicament, to promote and modulate the inflammatory phenomena of reparation of a tissue damage.

## Claims

1. An implant device for the implant in animal or human body, comprising a hyaluronic acid coating, **characterized in that** said hyaluronic acid is composed of a mixture of low molecular weight hyaluronic acid and high molecular weight hyaluronic acid, in which said low molecular weight hyaluronic acid has a molecular weight lower than 80,000 Dalton and said high molecular weight hyaluronic acid has a molecular weight higher than 700,000 Dalton, wherein said hyaluronic acid is chemically linked to the surface of said implant device.

2. The implant device according to claim 1, wherein said low molecular weight hyaluronic acid has a molecular weight lower than 30,000 Dalton.

3. The implant device according to claim 1, wherein said low molecular weight hyaluronic acid has a molecular weight lower than 19,000 Dalton or lower than 15,000 Dalton.

4. The implant device according to claim 1, wherein said low molecular weight hyaluronic acid has a molecular weight lower than 10,000 Dalton or lower than 7,000 Dalton and higher than 1,000 Dalton.

5. The implant device according to any claim 1 to 4, wherein said high molecular weight hyaluronic acid has a molecular weight higher than 1,000,000 Dalton.

6. The implant device according to any claim 1 to 4, wherein said high molecular weight hyaluronic acid has a molecular weight higher than 1,500,000 Dalton.

7. The implant device according to any claim 1 to 4, wherein said high molecular weight hyaluronic acid has a molecular weight higher than 2,000,000 Dalton and lower than 3,000,000 Dalton.

8. The implant device according to any claim 1 to 7, wherein, in said mixture of low and high molecular weight hyaluronic acid, the low molecular weight hyaluronic acid is present in a weight percentage ranging between 10% and 90%, or between 20% and the 80%, or between 25% and 75% or between 50% and 65%.

9. The implant device according to any claim 1 to 7, wherein, in said mixture of low and high molecular weight hyaluronic acid, low molecular weight hyaluronic acid is present in a weight percentage ranging between 10% and 50%, or between 15% and 45%, or between 25% and 40%.

10. The implant device according to any claim 1 to 9, wherein said device is a orthopaedic implant such as an osteo-implant, a screw for dental implant, also of the trans-mucosal type, a screw for spinal or skeletal fixing, an intervertebral disc, a cardiovascular stent, both of coronary and peripheral type, a "drug eluting stent", or a balloon for catheters.

11. The implant device according to any claim 1 to 10, comprising a coating of a substrate having amino groups, wherein said hyaluronic acid is linked to said substrate.

12. The implant device according to claim 11, wherein said substrate having amino groups comprises allylamine or alkylammine units, preferably selected from hexyl or heptyl amine.

13. The implant device according to claim 11, wherein said substrate having amino groups comprises or is composed of polyethyleneimine.

14. The implant device according to claim 11, wherein said substrate having amino groups comprises or is composed of type I collagen.

15. The implant device according to any claim 1 to 14, wherein said hyaluronic acid layer has a thickness ranging between 0.5 and 10000 nm.

16. The implant device according to claim 15, wherein said hyaluronic acid layer has a thickness ranging between 1.5 and 100 nm.

17. The implant device according to any claim 10 to 16, said device being a orthopaedic implant such as an osteo-implant comprising a mixture wherein said low molecular weight hyaluronic acid is present in amounts ranging between 20% and 30% by weight and said high molecular weight hyaluronic acid is present in amounts ranging between 80% and 70% by weight, and wherein said low molecular weight hyaluronic acid has a molecular weight ranging between 10,000 and 15,000 Dalton and said high molecular weight hyaluronic acid has a molecular weight ranging between 700,000 and 1,000,000 Dalton.

18. The implant device according to any claim 10 to 16, said device being a vascular stent comprising a coating wherein said low molecular weight hyaluronic acid is present in amounts ranging between 35% and 45% and said high molecular weight hyaluronic acid is present in amounts ranging between 65% and 55% by weight, and wherein said low molecular weight hyaluronic acid has a molecular weight ranging between 30,000 and 80,000 Dalton and said high molecular weight hyaluronic acid has a molecular weight higher than 2,000,000 Dalton and lower than 3,000,000 Dalton.

19. A mixture of hyaluronic acid with molecular weight lower than 80,000 Dalton and hyaluronic acid with molecular weight higher than 700,000 Dalton, for use in promoting and modulating the inflammatory phenomena of reparation of a tissue damage, when the said hyaluronic acid is immobilized on an implant device.

20. Hyaluronic acid according to claim 19, wherein said implant device is as set forth in any claim 1 to 16.

## Patentansprüche

1. Implantatvorrichtung zur Implantation in einen tierischen oder menschlichen Körper, umfassend eine Hyaluronsäurebeschichtung, **dadurch gekennzeichnet, dass** die Hyaluronsäure zusammengesetzt ist aus einem Gemisch aus Hyaluronsäure mit geringem Molekulargewicht und Hyaluronsäure mit hohem Molekulargewicht, worin die Hyaluronsäure mit geringem Molekulargewicht ein Molekulargewicht von weniger als 80000 Dalton aufweist und die Hyaluronsäure mit hohem Molekulargewicht ein Molekulargewicht von mehr als 700000 Dalton aufweist, worin die Hyaluronsäure chemisch an die Oberfläche der Implantatvorrichtung gebunden ist.

2. Implantatvorrichtung nach Anspruch 1, worin die Hyaluronsäure mit geringem Molekulargewicht ein Molekulargewicht von weniger als 30000 Dalton aufweist.

3. Implantatvorrichtung nach Anspruch 1, worin die Hyaluronsäure mit geringem Molekulargewicht ein Molekulargewicht von weniger als 19000 Dalton oder weniger als 15000 Dalton aufweist.

4. Implantatvorrichtung nach Anspruch 1, worin die Hyaluronsäure mit geringem Molekulargewicht ein Molekulargewicht von weniger als 10000 Dalton oder weniger als 7000 Dalton und höher als 1000 Dalton aufweist.

5. Implantatvorrichtung nach einem der Ansprüche 1 bis 4, worin die Hyaluronsäure mit hohem Molekulargewicht ein Molekulargewicht von mehr als 1000000 Dalton aufweist.

6. Implantatvorrichtung nach einem der Ansprüche 1 bis 4, worin die Hyaluronsäure mit hohem Molekulargewicht ein Molekulargewicht von mehr als 1500000 Dalton aufweist.

7. Implantatvorrichtung nach einem der Ansprüche 1 bis 4, worin die Hyaluronsäure mit hohem Molekulargewicht ein Molekulargewicht von mehr als 2000000 Dalton und weniger als 3000000 Dalton aufweist.

8. Implantatvorrichtung nach einem der Ansprüche 1 bis 7, worin in dem Gemisch aus Hyaluronsäure mit geringem und hohem Molekulargewicht, die Hyaluronsäure mit geringem Molekulargewicht in einem Gewichtsprozentanteil im Bereich zwischen 10% und 90% oder zwischen 20% und 80% oder zwischen 25% und 75% oder zwischen 50% und 65% vorliegt.

9. Implantatvorrichtung nach einem der Ansprüche 1 bis 7, worin in dem Gemisch aus Hyaluronsäure mit geringem und hohem Molekulargewicht, die Hyaluronsäure mit geringem Molekulargewicht in einem Gewichtsprozentanteil im Bereich zwischen 10% und 50% oder zwischen 15% und 45, oder zwischen 25% und 40% vorliegt.

10. Implantatvorrichtung nach einem der Ansprüche 1 bis 9, worin die Vorrichtung ein orthopädisches Implantat ist, wie etwa ein Osteo-Implantat, eine Schraube für Dentalimplantat, auch vom transmukosalen Typ, eine Schraube für Spinal- oder Skelettfixation, eine Bandscheibe, ein kardiovaskulärer Stent, sowohl koronarer- als auch periphärer Typ, ein "Arzneimittel-eluierender Stent" oder ein Ballon für Katheter.

11. Implantatvorrichtung nach einem der Ansprüche 1 bis 10, umfassend eine Beschichtung eines Substrats mit Aminogruppen, worin die Hyaluronsäure an das Substrat gebunden wird.

12. Implantatvorrichtung nach Anspruch 11, worin das Substrat mit Aminogruppen Allylamin- oder Alkylamineinheiten, vorzugsweise ausgewählt aus Hexyl- oder Heptylamin, umfasst.

13. Implantatvorrichtung nach Anspruch 11, worin das Substrat mit Aminogruppen Polyethylenimin umfasst oder aus Polyethylenimin aufgebaut ist.

14. Implantatvorrichtung nach Anspruch 11, worin das Substrat mit Aminogruppen Typ I-Collagen umfasst oder aus Typ I-Collagen aufgebaut ist.

15. Implantatvorrichtung nach einem der Ansprüche 1 bis 14, worin die Hyaluronsäureschicht eine Dicke im Bereich zwischen 0,5 und 10000 nm aufweist.

16. Implantatvorrichtung nach Anspruch 15, worin die Hyaluronsäureschicht eine Dicke im Bereich zwischen 1,5 und 100 nm aufweist.

17. Implantatvorrichtung nach einem der Ansprüche 10 bis 16, worin die Vorrichtung ein orthopädisches Transplantat ist, wie etwa ein Osteo-Implantat, umfassend ein Gemisch, worin die Hyaluronsäure mit geringem Molekulargewicht in Mengen vorliegt im Bereich zwischen 20% und 30% bezüglich des Gewichts und die Hyaluronsäure mit hohem Molekulargewicht in Mengen im Bereich zwischen 80% und 70% bezüglich des Gewichts vorliegt, und worin die Hyaluronsäure mit geringem Molekulargewicht ein Molekulargewicht im Bereich zwischen 10000 und 15000 Dalton aufweist und die Hyaluronsäure mit hohem Molekulargewicht ein Molekulargewicht im Bereich zwischen 700000 und 1000000 Dalton aufweist.

18. Implantatvorrichtung nach einem der Ansprüche 10 bis 16, worin die Vorrichtung ein vaskulärer Stent ist, umfassend eine Beschichtung, worin die Hyaluronsäure mit geringem Molekulargewicht in Mengen im Bereich zwischen 35% und 45% vorliegt und die Hyaluronsäure mit hohem Molekulargewicht in Mengen im Bereich zwischen 65% und 55% bezüglich des Gewichts vorliegt, und worin die Hyaluronsäure mit geringem Molekulargewicht ein Molekulargewicht im Bereich zwischen 30000 und 80000 Dalton aufweist und die Hyaluronsäure mit hohem Molekulargewicht ein Molekulargewicht von höher als 2000000 Dalton und weniger als 3000000 Dalton aufweist.

19. Gemisch aus Hyaluronsäure mit einem Molekulargewicht von weniger als 80000 Dalton und einer Hyaluronsäure mit einem Molekulargewicht von höher als 700000 Dalton zur Verwendung beim Unterstützen und Modulieren der Entzündungsphänomene bei der Reparatur von Gewebeschädigung wenn die Hyluronsäure auf einer Implantatvorrichtung immobilisiert ist.

20. Hyaluronsäure nach Anspruch 19, worin die Implantatvorrichtung wie in einem der Ansprüche 1 bis 16 angegeben, ist.

## Revendications

1. Dispositif d'implant pour l'implant dans un corps animal ou humain, comprenant un revêtement d'acide hyaluronique, **caractérisé en ce que** ledit acide hyaluronique est composé d'un mélange d'acide hyaluronique de faible masse moléculaire et d'acide hyaluronique de haute masse moléculaire, où ledit acide hyaluronique de faible masse moléculaire a une masse moléculaire inférieure à 80 000 daltons et ledit acide hyaluronique à haute masse moléculaire a une masse moléculaire supérieure à 700 000 daltons, où ledit acide hyaluronique est lié chimiquement à la surface dudit dispositif d'implant.

2. Dispositif d'implant selon la revendication 1, où ledit acide hyaluronique de faible masse moléculaire a une masse moléculaire inférieure à 30 000 daltons.

3. Dispositif d'implant selon la revendication 1, où ledit acide hyaluronique de faible masse moléculaire a une masse moléculaire inférieure à 19 000 daltons ou inférieure à 15 000 daltons.

4. Dispositif d'implant selon la revendication 1, où ledit acide hyaluronique de faible masse moléculaire a une masse moléculaire inférieure à 10 000 daltons ou inférieure à 7 000 daltons et supérieure à 1 000 daltons.

5. Dispositif d'implant selon l'une quelconque des revendications 1 à 4, où ledit acide hyaluronique de haute masse moléculaire a une masse moléculaire supérieure à 1 000 000 de daltons.

6. Dispositif d'implant selon l'une quelconque des revendications 1 à 4, où ledit acide hyaluronique de haute masse moléculaire a une masse moléculaire supérieure à 1 500 000 daltons.

7. Dispositif d'implant selon l'une quelconque des revendications 1 à 4, où ledit acide hyaluronique de haute masse moléculaire a une masse moléculaire supérieure à 2 000 000 de daltons et inférieure à 3 000 000 de daltons.

8. Dispositif d'implant selon l'une quelconque des revendications 1 à 7, où, dans ledit mélange d'acide hyaluronique de faible masse moléculaire et de haute masse moléculaire, l'acide hyaluronique de faible masse moléculaire est présent en un pourcentage en poids situé entre 10 % et 90 % ou entre 20 % et 80 % ou entre 25 % et 75 % ou entre 50 % et 65 %.

9. Dispositif d'implant selon l'une quelconque des revendications 1 à 7, où, dans ledit mélange d'acide hyaluronique de faible masse moléculaire et de haute masse moléculaire, l'acide hyaluronique de faible masse moléculaire est présent en un pourcentage en poids situé entre 10 % et 50 % ou entre 15 % et 45 % ou entre 25 % et 40 %.

10. Dispositif d'implant selon l'une quelconque des revendications 1 à 9, où ledit dispositif est un implant orthopédique tel qu'un ostéo-implant, une vis pour implant dentaire, également du type trans-muqueux, une vis pour fixation spinale ou squelettique, un disque intervertébral, un stent cardiovasculaire, l'un et l'autre de type coronaire et périphérique, un "stent à élution de médicament", ou un ballonnet pour cathéters.

11. Dispositif d'implant selon l'une quelconque des revendications 1 à 10 comprenant un revêtement d'un substrat ayant des groupes amino, où ledit acide hyaluronique est lié audit substrat.

12. Dispositif d'implant selon la revendication 11, où ledit substrat ayant des groupes amino comprend des unités d'allylamine ou d'alkylamine, de préférence choisies parmi l'hexyl ou heptylamine.

13. Dispositif d'implant selon la revendication 11, où ledit substrat ayant des groupes amino comprend ou est composé de polyéthylèneimine.

14. Dispositif d'implant selon la revendication 11, où ledit substrat ayant des groupes amino comprend ou est composé de collagène de type I.

15. Dispositif d'implant selon l'une quelconque des revendications 1 à 14, où ladite couche d'acide hyaluronique a une épaisseur située entre 0,5 et 10 000 nm.

16. Dispositif d'implant selon la revendication 15, où ladite couche d'acide hyaluronique a une épaisseur située entre 1,5 et 100 nm.

17. Dispositif d'implant selon l'une quelconque des revendications 10 à 16, ledit dispositif étant un implant orthopédique tel qu'un ostéo-implant comprenant un mélange où ledit acide hyaluronique de faible masse moléculaire est présent en des quantités situées entre 20 % et 30 % en poids et ledit acide hyaluronique de haute masse moléculaire est présent en des quantités situées entre 80 % et 70 % en poids, et où ledit acide hyaluronique de faible masse moléculaire a une masse moléculaire située entre 10 000 et 15 000 daltons et ledit acide hyaluronique de haute masse moléculaire a une masse moléculaire située entre 700 000 et 1 000 000 de daltons.

18. Dispositif d'implant selon l'une quelconque des revendications 10 à 16, ledit dispositif étant un stent vasculaire comprenant un revêtement où ledit acide hyaluronique de faible masse moléculaire est présent en des quantités situées entre 35 % et 45 % et ledit acide hyaluronique de haute masse moléculaire est présent en des quantités situées entre 65 % et 55 % en poids, et où ledit acide hyaluronique de faible masse moléculaire a une masse moléculaire située entre 30 000 et 80 000 daltons et ledit acide hyaluronique à haute masse moléculaire a une masse moléculaire supérieure à 2 000 000 de daltons et inférieure à 3 000 000 de daltons.

19. Mélange d'acide hyaluronique ayant une masse moléculaire inférieure à 80 000 daltons et d'acide hyaluronique ayant une masse moléculaire supérieure à 700 000 daltons, destiné à être utilisé pour favoriser et moduler les phénomènes inflammatoires de réparation d'une lésion tissulaire, quand ledit acide hyaluronique est immobilisé sur un dispositif d'implant.

20. Acide hyaluronique selon la revendication 19, où ledit dispositif d'implant est tel que présenté dans l'une quelconque des revendications 1 à 16.
